# EUROPÄISCHE PATENTSCHRIFT

(11) **EP 0 443 392 B1**
(45) Veröffentlichungstag und Bekanntmachung des Hinweises auf die Patenterteilung: **09.08.1995**
(21) Anmeldenummer: 91101833.1
(22) Anmeldetag: 09.02.1991
(51) Int. Cl.: C07D 307/08, C07D 307/32

(54) **Verfahren zur Herstellung von Tetrahydrofuran und alpha-Butyrolacton**
Process for the production of tetrahydrofuran and alphabutyrolactone
Procédé de fabrication de tétrahydrofuranne et alphabutyrolactone

(30) Priorität: 20.02.1990 DE 4005293
(43) Veröffentlichungstag der Anmeldung: 28.08.1991
(73) Patentinhaber: BASF Aktiengesellschaft, 67063 Ludwigshafen (DE)
(72) Erfinder: Zimmermann, Horst, Dr., W-6800 Mannheim 1 (DE); Brenner, Karl, W-6700 Ludwigshafen (DE); Scheiper, Hans-Jürgen, W-6704 Mutterstadt (DE); Sauer, Wolfgang, Dr., W-6719 Kirchheimbolanden (DE); Hartmann, Horst, W-6737 Boehl-Iggelheim (DE)

(56) Entgegenhaltungen:
- WO-A-86/03189
- DE-A- 1 953 583
- DE-A- 2 704 500
- DE-B- 1 043 342

## Beschreibung

Die vorliegende Erfindung betrifft ein Verfahren zur Herstellung von Tetrahydrofuran oder von Tetrahydrofuran und γ-Butyrolacton aus dem Hydrierprodukt der Hydrierung von Maleinsäure, Bernsteinsäure, Maleinsäureanhydrid, Bernsteinsäureanhydrid und/oder Fumarsäure.

Im folgenden Text werden die folgenden Kurzbezeichnungen verwendet: MS für Maleinsäure, BS für Bernsteinsäure, MSA für Maleinsäureanhydrid, BSA für Bernsteinsäureanhydrid, THF für Tetrahydrofuran, GBL für γ-Butyrolacton, BD für 1,4-Butandiol, FS für Fumarsäure.

Die säurekatalysierte Cyclisierung von BD zu THF ist bekannt, beispielsweise aus DE-A 34 32 575 und DE-A 29 30 144.

Die Hydrierung der genannten Ausgangsstoffe zu Gemischen aus THF, GBL und/oder BD ist bekannt, beispielsweise aus US-A 4 155 919, US-A 4 096 156, DE-A 21 33 768, EP-A 184 055, DE-A 37 26 510, EP-A 304 696 und US-A 4 751 334. Dabei entstehen THF, GBL und BD je nach verwendetem Katalysator und in Abhängigkeit von den angewandten Hydrierbedingungen als Hauptprodukte in unterschiedlichen Mengenverhältnissen. Des weiteren entstehen noch eine Vielzahl von Nebenprodukten, beispielsweise Dibutylenglykol, 4-Hydroxybuttersäureester, Buttersäure-(4-hydroxybutyl)ester, Bernsteinsäure-(4-hydroxybutyl)ester, Butoxybutanol usw.

Die nach diesem verfahren erhaltenen Hydriergemische werden destillativ aufgearbeitet. Infolge der im Hydrierprodukt enthaltenen Nebenprodukte gestaltet sich diese Aufarbeitung schwierig und erfordert einen hohen Aufwand an Apparaten und Energie. Dies gilt insbesondere dann, wenn reines THF oder GBL gewonnen werden soll. Befindet sich im Hydrieraustrag noch BD, ist dieses vom THF und GBL und gegebenenfalls noch von weiteren im Hydrieraustrag vorliegenden Produkten abzutrennen, bevor es durch Cyclisierung in THF umgewandelt werden kann.

So wird in US-A 4 751 334 ein verfahren beschrieben, welches bei der Hydrierung von Maleinsäureester ein Hydrierprodukt liefert, das im wesentlichen aus THF, GBL und BD besteht. Bei der destillativen Aufarbeitung des Hydriergemisches wird ein Leichtsiederanteil, bestehend aus THF, Alkoholen und niedrigsiedenden Verunreinigungen, über Kopf abdestilliert und das Sumpfprodukt in weiteren Destillationskolonnen in ein GBL-Diethylsuccinat-Azeotrop und rohes Butandiol zerlegt. Die in den jeweiligen Fraktionen enthaltenen Wertprodukte THF, GBL, bzw. BD sind noch stark verunreinigt und müssen anschließend nochmals in separaten Destillationsanlagen aufgearbeitet werden. Das von THF und GBL abgetrennte BD kann nach diesem Verfahren gewünschtenfalls in einer weiteren Cyclisierungsstufe zu THF dehydratisiert werden.

In US-A 4 851 085 ist ein Verfahren zur Entfernung von Verfärbungen bildenden Verunreinigungen aus GBL beschrieben, bei dem das GBL mit einer Säure behandelt wird. Eine notwendige Voraussetzung für den Erfolg dieses Verfahrens ist die Verwendung von GBL einer Reinheit von mindestens 95 %.

DE-A 19 53 583 beschreibt ein Gasphasenverfahren zur Herstellung von Tetrahydrofuran, in dem Maleinsäureanhydrid, Maleinsaure und deren Ester, Bernsteinsäureanhydrid, Bernsteinsäure und deren Ester, γ-Butyrolacton oder Mischungen dieser Verbindungen mit Wasserstoff in einer Reaktionszone umgesetzt werden, die mit einer Katalysatormischung aus einem kupferhaltigen Hydrierkatalysator und einem Wasserabspaltungskatalysator beschickt ist.

Infolge des hohen Aufwandes zur Abtrennung, Isolierung und Reinigung der Wertprodukte THF, GBL und BD sind die Verfahren zur Herstellung von THF und GBL durch die Hydrierung von Maleinsäure und deren Derivaten unwirtschaftlich. Der vorliegenden Erfindung lag daher die Aufgabe zugrunde, ein Verfahren zu entwickeln, das die wirtschaftliche Herstellung und Gewinnung von THF und GBL aus dem Hydrierprodukt der Maleinsäurehydrierung erlaubt und das insbesondere die Herstellung und Gewinnung von reinem THF und GBL mit einem erheblich verminderten Reinigungsaufwand ermöglicht.

Dementsprechend wurde ein Verfahren zur Herstellung von THF oder THF und γ-Butyrolacton aus dem Hydrierprodukt der Hydrierung von Maleinsäure, Bernsteinsäure, Maleinsäureanhydrid, Bernsteinsäureanhydrid und/oder Fumarsäure, gefunden, das dadurch gekennzeichnet ist, daß man das rohe Hydrierprodukt ohne vorherige Aufarbeitung bei 100 bis 300°C mit Protonensäuren behandelt und aus der erhaltenen Reaktionsmischung reines Tetrahydrofuran und γ-Butyrolacton destillativ isoliert.

Unter dem Begriff Hydrierprodukt werden die BD oder die BD und THF als auch die BD, THF und GBL als wertgebende Bestandteile enthaltenden Hydrierausträge der katalytischen Hydrierung von MS, BS, FS, MSA und/oder BSA verstanden.

Mit Hilfe des erfindungsgemäßen Verfahrens, nämlich der Säurebehandlung des Hydrieraustrages, gelingt es überraschenderweise praktisch quantitativ das im Hydrieraustrag befindliche BD in THF und die im Hydrieraustrag befindlichen Nebenprodukte säurekatalysiert in THF oder GBL umzuwandeln.

Diejenigen Nebenprodukte, welche nicht in THF oder GBL umwandelbar sind, werden unerwarteterweise unter den Reaktionsbedingungen der erfindungsgemäßen Säurebehandlung offensichtlich zu solchen Produkten zersetzt, die die destillative Aufarbeitung der Wertprodukte THF und GBL nicht stören, so daß THF, beispielsweise als THF-Wasser-Azeotrop, als auch GBL auf einfache Weise in besonders reiner Form erhalten werden können. Das entstandene THF-Wasser-Azeotrop kann nach Standardmethoden, beispielsweise dem Verfahren der DE-A 37 26 805 in seine Bestandteile THF und Wasser zerlegt werden.

Zur erfindungsgemäßen Säurebehandlung des Hydrieraustrags können eine Vielzahl von Protonensäuren verwendet werden: beispielsweise können herkömmliche anorganische Protonensäuren wie Salzsäure, Schwefelsäure, Phosphorsäure oder Tetrafluoroborsäure als auch organische Protonensäuren wie Oxalsäure, p-Toluolsulfonsäure oder Methansulfonsäure verwendet werden. Praktisch können alle Protonensäuren verwendet werden, welche genügend acide sind, um die Dehydratisierung von BD zu katalysieren und unter den Reaktionsbedingungen nicht oxidierend wirken, insbesondere nichtoxidierende Mineralsäuren. Somit können im erfindungsgemäßen Verfahren beispielsweise auch Heteropolysäuren wie Wolframatophosphorsäuren, Wolframatokieselsäuren oder Molybdatophosphorsäuren benutzt werden.

Außer den genannten herkömmlichen Protonensäuren können auch Feststoffe verwendet werden, die acide sind und sich wie Brönstedt-Säuren verhalten. Beispielsweise seien dehydratisierend wirkende, acide Oxide des Aluminiums, Magnesiums, Titans und Zirkoniums, Aluminiumphosphate, Zeolithe wie Faujasite, Pentasile oder Silicalite sowie Schichtsilikate wie Montmorillonite, Bleicherden wie Bentonite, Fuller-Erden usw. genannt. Selbstverständlich können auch organische Kationenaustauscher, beispielsweise sulfonierte Phenol-Formaldehyd-Harze, sulfonierte Styrol-Divinylbenzol-Copolymere, Polyacrylate, Kationenaustauscher auf Cellulose-Basis oder sulfonierte Kohlen als acide Katalysatoren angewandt werden.

Bevorzugt werden Schwefelsäure, Phosphorsäure sowie Schichtsilikate, insbesondere Bentonit und organische Kationenaustauscher verwendet. Besonders vorteilhaft ist die Verwendung von Schwefelsäure.

Zur Säurebehandlung wird der Hydrieraustrag im allgemeinen mit 0,01 bis 10 Gew.-%, vorteilhaft 0,01 bis 5 Gew.-% und besonders bevorzugt mit 0,1 bis 1 Gew.-% der betreffenden Säure versetzt. Die Protonensäuren können dem Hydrieraustrag als wäßrige Lösung oder falls dies für die jeweilige Säure möglich ist, gasförmig - z.B. Chlorwasserstoff - oder in fester Form - z.B. p-Toluolsulfonsäure, Bentonit, Wolframatophosphorsäure - zugeführt werden. Die flüssigen Protonensäuren, beispielsweise Schwefelsäure oder Phosphorsäure, werden in der Regel in Form ihrer handelsüblichen, konzentrierten Lösungen verwendet, doch ist auch, bei entsprechend höheren Aufwandmengen, die Verwendung verdünnter Lösungen dieser Säuren möglich. Selbstverständlich müssen die verdünnten Lösungen dieser Säuren noch ausreichend acide sein, um die Cyclisierung von BD zu THF effektiv katalysieren zu können. Vorzugsweise werden jedoch die konzentrierten Säuren verwendet.

Die Säurebehandlung des Hydrieraustrags wird im allgemeinen bei Temperaturen von 100 bis 300°C vorgenommen. Dabei ist die letztendlich vorteilhaft einzustellende Reaktionstemperatur auch von der Art der verwendeten Säure abhängig und kann jeweils mittels einiger weniger Vorversuche festgelegt werden. Beispielsweise wird die Säurebehandlung mit Schwefelsäure vorteilhaft bei 100 bis 150, insbesondere bei 110 bis 130°C durchgeführt, wohingegen bei Verwendung von Bentonit vorzugsweise Reaktionstemperaturen von 150 bis 200, insbesondere von 170 bis 190°C, eingestellt werden und bei der Behandlung mit Phosphorsäure sind im allgemeinen Reaktionstemperaturen von 250 bis 300°C vorteilhaft.

Die Säurebehandlung kann sowohl unter Atmosphärendruck als auch unter erhöhtem Druck, vorzugsweise unter dem Eigendruck des Reaktionssystems durchgeführt werden. Da die betreffenden Hydrierausträge in den meisten Fällen bereits größere Mengen THF enthalten, ist es möglich, einhergehend mit der Säurebehandlung den Leichtsiederanteil der Reaktionsmischung, bestehend aus einem THF-Wasser-Azeotrop, destillativ abzutrennen. Dabei wird zu Beginn hauptsächlich das bereits im Hydrieraustrag vorhandene THF abgezogen, während im Laufe der weiteren Behandlung der Anteil des aus BD neu gebildeten Tetrahydrofurans im Destillat zunimmt. Da sich das bereits im Hydrieraustrag vorhandene THF unter den Bedingungen der Säurebehandlung praktisch inert verhält, ist die destillative Entfernung des Tetrahydrofurans aus dem Hydrieraustrag vor dessen Säurebehandlung der Entfernung des Tetrahydrofurans während der Säurebehandlung als äquivalent zu betrachten.

Die Säurebehandlung des Hydrieraustrages kann diskontinuierlich in gegebenenfalls druckstabilen Rührkesseln oder kontinuierlich, beispielsweise in Rührkesselkaskaden, vorgenommen werden. Werden feste Cyclisierungskatalysatoren, beispielsweise Bleicherden, verwendet, so kann der Katalysator im Reaktionsmedium suspendiert werden, vorteilhaft wird aber eine Festbettanordnung des Katalysators gewählt, über die der betreffende Hydrieraustrag in der Sumpf- oder Rieselfahrweise geleitet wird.

Da im Zuge der Säurebehandlung das bereits im Hydrieraustrag vorhandene als auch das sich neu bildende THF kontinuierlich aus der Reaktionsmischung abdestilliert werden kann, verbleibt am Ende der Säurebehandlung in der Reaktionsapparatur ein schwerflüchtiger Rückstand, der im wesentlichen aus GBL und noch schwerer flüchtigen Verbindungen besteht, von denen GBL auf einfache Weise destillativ abgetrennt und in einer Reinheit von mehr als 99 % erhalten werden kann. Die Katalysatorsäure bleibt im Destillationsrückstand zurück und kann gewünschtenfalls regeneriert werden. Somit ermöglicht das erfindungsgemäße Verfahren die sukzessive Gewinnung von hochreinem THF und GBL aus dem Hydrieraustrag der MSA-Hydrierung in ein- und derselben Anlage.

Die Hydrierverfahren zur Gewinnung der betreffenden Hydrierausträge sind nicht Gegenstand der vorliegenden Erfindung. In das erfindungsgemäße Verfahren können die Hydrierausträge aller Verfahren zur Hydrierung von MS, MSA, BS, BSA oder FS, insbesondere die Hydrierausträge der eingangs erwähnten Hydrierverfahren, eingesetzt werden. Da die Hydrierausträge der verschiedenen Hydrierverfahren unterschiedlicher Zusammensetzung sind, entstehen bei der erfindungsgemäßen Behandlung der verschiedenen Hydrierausträge THF und GBL selbstverständlich in unterschiedlichen Anteilen. Beispielsweise kann aus dem Hydrieraustrag des Verfahrens von US-A 4 155 919 erfindungsgemäß praktisch nur THF gewonnen werden, wohingegen aus dem Hydrieraustrag des Verfahrens von EP-A 304 696 erfindungsgemäß außer THF noch beträchtliche Mengen an GBL erhalten werden können. Vorzugsweise werden im erfindungsgemäßen Verfahren solche Hydrierausträge verwendet, welche durch die Hydrierung von Schmelzen von MS, MSA, BS, BSA oder FS erhalten worden sind.

Das nach dem erfindungsgemäßen Verfahren gewonnene GBL als auch das erfindungsgemäß nach Zerlegung des THF-Wasser-Azeotrops erhaltene THF sind so rein, daß sie zu den allermeisten Verwendungszwecken ohne weitere Reinigung benutzt werden können.

THF und GBL sind wichtige Lösungsmittel und dienen außerdem als Zwischenprodukte zur Herstellung von Poly-THF (Polytetramethylenglykol) bzw. N-Methylpyrrolidon.

### Beispiele

### Beispiel 1

Ein typischer Austrag der MSA-Hydrierung mit einer Zusammensetzung gemäß Tabelle 1 wurde mit 0,1 Gew.-% konzentrierter Schwefelsäure versetzt und mehrere Stunden auf 130°C erhitzt. Während dieser Zeit wurde das bereits im Hydrieraustrag enthaltene THF und Wasser zusammen mit dem im Zuge der Umsetzung neu gebildeten THF und Wasser kontinuierlich als THF-Wasser-Azeotrop aus dem Reaktionsgemisch abdestilliert. Nach der vollständigen Cyclisierung des Butandiols und der Zersetzung der Nebenprodukte wurde die Temperatur auf 110°C erniedrigt, der Druck auf 10 mbar abgesenkt und GBL abdestilliert. Das so erhaltene GBL hatte eine Reinheit von größer 99 %. Der Destillationsrückstand wurde nicht weiter aufgearbeitet und entsorgt. Das THF-Wasser-Azeotrop wurde nach dem Verfahren von DE-A 37 26 805 in seine beiden Bestandteile zerlegt, wobei ein THF mit einer Reinheit von größer 99,95 % erhalten wurde.

Tabelle 2 gibt die Produktverteilung nach der Säurebehandlung des Hydrieraustrages wieder.

**Tabelle 1**

| Zusammensetzung des Hydrieraustrages | |
|---|---|
| H₂O | 26,4 % |
| THF | 36,45 % |
| Propanol | 0,72 % |
| Butanol | 1,53 % |
| Butyrolacton | 13,63 % |
| Butandiol | 17,91 % |
| Rest | 3,36 % |

**Tabelle 2**

| Produktverteilung nach der Säurebehandlung | |
|---|---|
| H₂O | 30,30 % |
| THF | 52,5 % |
| Propanol | 0,75 % |
| Butanol | 1,68 % |
| Butyrolacton | 14,46 % |
| Butandiol | - |
| Rest | 0,31 % |

### Beispiel 2

Der in Beispiel 1 verwendete Hydrieraustrag wurde mit 0,2 Gew.-% Bentonit K10 versetzt und mehrere Stunden bei 185°C analog Beispiel 1 behandelt und aufgearbeitet. Es wurden praktisch die gleichen Ergebnisse wie in Beispiel 1 erzielt.

### Beispiel 3

Der in Beispiel 1 verwendete Hydrieraustrag wurde mit 0,2 Gew.-% konzentrierter Phosphorsäure in einem Autoklaven unter Eigendruck bei 260°C umgesetzt. Das Butandiol dehydratisierte vollständig zu THF.

### Beispiel 4

Der in Beispiel 1 verwendete Hydrieraustrag wurde mit 0,2 Gew.-% des in der H^{⊕}-Form befindlichen Kationenaustauschers Lewatit® SPC 118 bei 140°C analog dem Verfahren von Beispiel 1 behandelt und aufgearbeitet. Der Umsatz war vollständig. Mit Hilfe der Säurebehandlung konnte aus dem Hydrieraustrag mehr THF erzeugt werden als nach dem Gehalt des Hydrieraustrags an BD zu erwarten gewesen wäre. Dies ist vermutlich auf die säurekatalysierte Zersetzung von im Hydrieraustrag enthaltenen Nebenprodukten zurückzuführen. GBL wurde in der erwarteten Menge erhalten.

### Beispiel 5

Der in Beispiel 1 verwendete Hydrieraustrag wurde mit 0,2 Gew.-% fester Wolframatophosphorsäure bei 150°C analog dem Verfahren von Beispiel 1 umgesetzt und aufgearbeitet. THF und GBL wurden in praktisch quantitativer Ausbeute erhalten. Der Umsatz war vollständig.

### Beispiel 6

Ein typischer, nahezu GBL-freier Hydrieraustrag der Zusammensetzung

| | |
|---|---|
| Wasser | 28,9 Gew.-% |
| THF | 35,8 Gew.-% |
| BD | 29,3 Gew.-% |
| Propanol | 0,9 Gew.-% |
| Butanol | 1,7 Gew.-% |
| GBL | 0,1 Gew.-% |
| Rest | 3,3 Gew.-% |

wurde analog Beispiel 1 mit Schwefelsäure behandelt und aufgearbeitet. Die Produktverteilung nach der Säurebehandlung war folgendermaßen:

| | |
|---|---|
| Wasser | 34,98 Gew.-% |
| THF | 61,94 Gew.-% |
| BD | - |
| Propanol | 0,9 Gew.-% |
| Butanol | 1,76 Gew.-% |
| GBL | 0,1 Gew.-% |
| Rest | 0,29 Gew.-% |

### Beispiel 7 (Vergleichsbeispiel)

Ein Austrag aus der MSA-Hydrierung mit der Zusammensetzung gemäß Tabelle 1 wurde nach dem verfahren der US-A 4 751 334 destillativ in die Produkte THF, GBL und BD getrennt.

Zu diesem Zweck wurde in einer ersten Kolonne THF und Wasser abdestilliert und das THF-Wasser-Azeotrop gemäß DE-A 37 26 805 zu reinem THF aufgearbeitet. Im Sumpf dieser ersten Kolonne fielen dabei neben Wasser, Propanol, Butanol, GBL und BD auch die Mittelsieder und Hochsieder an. In der zweiten Kolonne wurde das Wasser zusammen mit organischen Nebenprodukten über Kopf abdestilliert. Das Sumpfprodukt der zweiten Kolonne wurde der dritten Kolonne - der GBL-Vorlauf-Kolonne - und anschließend der vierten Kolonne - der GBL-Reinkolonne - zugeleitet. Das in dieser Kolonne erhaltene GBL hatte eine Reinheit von ca. 98 %. Die Hauptverunreinigung war Butoxybutanol mit einem Anteil von ca. 1 %.

Das Sumpfprodukt der vierten Kolonne wurde von einer fünften Kolonne zur Abtrennung des BD-Vorlaufs und in einer sechsten Kolonne zur BD-Reingewinnung destilliert. BD wurde unter diesen Bedingungen in einer Reinheit von ca. 96 % erhalten und war hauptsächlich durch GBL verunreinigt, welches sich im Sumpf der sechsten Kolonne durch die thermische Zersetzung von Hochsiedern gebildet hatte. Diese Verunreinigung ließ sich durch eine vorgeschaltete BD-Hochsieder-Trennung in einer weiteren Kolonne vermeiden. Das auf diese Weise erhaltene BD konnte nun auf bekannte Weise zu THF cyclisiert werden.

## Patentansprüche

1. Verfahren zur Herstellung von Tetrahydrofuran oder Tetrahydrofuran und γ-Butyrolacton aus dem Hydrierprodukt der Hydrierung von Maleinsäure, Bernsteinsäure, Maleinsäureanhydrid, Bernsteinsäureanhydrid und/oder Fumarsäure, dadurch gekennzeichnet, daß man das rohe Hydrierprodukt ohne vorherige Aufarbeitung bei 100 bis 300°C mit Protonensäuren behandelt und aus der erhaltenen Reaktionsmischung reines Tetrahydrofuran und γ-Butyrolacton destillativ isoliert.

2. Verfahren nach Anspruch 1, dadurch gekennzeichnet, daß man als Protonensäuren nichtoxidierende Mineralsäuren verwendet.

3. Verfahren nach Anspruch 1, dadurch gekennzeichnet, daß man als Protonensäuren, Protonensäure wirkende anorganische Feststoffe verwendet.

4. Verfahren nach Anspruch 1, dadurch gekennzeichnet, daß man als Protonensäuren saure Ionenaustauscher einsetzt.

## Claims

1. A process for the preparation of tetrahydrofuran or tetrahydrofuran and γ-butyrolactone from the product of the hydrogenation of maleic acid, succinic acid, maleic anhydride, succinic anhydride and/or fumaric acid, which comprises treating the crude hydrogenation product, without prior work-up, at from 100 to 300°C with a protic acid, and isolating pure tetrahydrofuran and γ-butyrolactone from the resultant reaction mixture by distillation.

2. A process as claimed in claim 1, wherein the protic acid used is a non-oxidizing mineral acid.

3. A process as claimed in claim 1, wherein the protic acid used is an inorganic solid which acts as a protic acid.

4. A process as claimed in claim 1, wherein the protic acid employed is an acidic ion exchanger.

## Revendications

1. Procédé de préparation du tétrahydrofuranne, ou du tétrahydrofuranne et de la γ-butyrolactone à partir du produit d'hydrogénation provenant de l'hydrogénation de l'acide maléique, de l'acide succinique, de l'anhydride maléique, de l'anhydride succinique et/ou de l'acide fumarique, caractérisé en ce que l'on traite le produit d'hydrogénation brut sans apprêtage préalable, à 100-300°C par des acides protoniques et en ce que l'on isole de la γ-butyrolactone et du tétrahydrofuranne pur par distillation du mélange réactionnel obtenu.

2. Procédé suivant la revendication 1, caractérisé en ce que l'on utilise, à titre d'acides protoniques, des acides minéraux non oxydants.

3. Procédé suivant la revendication 1, caractérisé en ce que l'on utilise, à titre d'acides protoniques, des solides inorganiques à activité d'acide protonique.

4. Procédé suivant la revendication 1, caractérisé en ce que l'on utilise, à titre d'acides protoniques, des échangeurs d'ions acides.
